# EUROPÄISCHE PATENTSCHRIFT

(11) **EP 2 848 611 B1**
(45) Veröffentlichungstag und Bekanntmachung des Hinweises auf die Patenterteilung: **25.05.2016**
(21) Anmeldenummer: 13183855.9
(22) Anmeldetag: 11.09.2013
(51) Int. Cl.: C07D 311/74, C07D 307/79

(54) **Octahydrobenzofurane und Octahydrochromene - Verfahren zur Herstellung und ihre Verwendung als Duftstoffe**
Octahydrobenzofurane and Octahydrochromene - Method for Preparation and Their Use as Fragrances
Octahydrobenzofurane et Octahydrochromene - Méthode de Préparation et Leur Utilisation Comme Parfums

(43) Veröffentlichungstag der Anmeldung: 18.03.2015
(73) Patentinhaber: Symrise AG, 37603 Holzminden (DE)
(72) Erfinder: Hoelscher, Bernd, 37620 Halle (DE); Panten, Johannes, 37671 Hoexter (DE)
(74) Vertreter: Miller, James Lionel Woolverton

(56) Entgegenhaltungen:
- WO-A1-2013/109837

## Beschreibung

### Gebiet der Erfindung

Die vorliegende Erfindung betrifft neue Duftstoffe, ein Verfahren zu ihrer Herstellung, Parfüms und Konsumgüter enthaltend diese Duftstoffe sowie deren Verwendungen.

### Stand der Technik

In der Parfümindustrie sind eine Vielzahl verschiedenster Verbindungen bekannt, die zur Vermittlung eines Wohlgeruchs eingesetzt werden. Es besteht der stetige Bedarf nach neuartigen vorteilhaften Verbindungen, die neben ihren positiven und originellen geruchlichen Eigenschaften zusätzliche positive Sekundäreigenschaften besitzen.

Bei der Suche nach geeigneten Verbindungen wir der Fachmann vor die schwierige Aufgabe gestellt, aus einer nahezu unendlich erscheinenden Anzahl möglicher Verbindungen, geeignete Duftstoffe auszuwählen. Dabei lässt sich nicht vorhersagen, ob unbekannte Verbindungen überhaupt einen Geruch aufweisen und ob dieser Geruch erwünschte oder unerwünschte geruchliche Eigenschaften aufweist. Beim Auffinden einer Verbindung mit positivem Geruch ist zudem sehr fraglich, um welchen Geruch es sich handelt und inwieweit die Verbindung auch über positive sekundäre Eigenschaften verfügt.

Viele bisher bekannte Duftstoffe weisen zum Teil deutliche Nachteile auf, beispielsweise besitzen diese eine sehr beschränkte Stabilität und Ausgiebigkeit, ein geringes Haftungsvermögen, eine geringe Ausstrahlungskraft, eine schlechte Löslichkeit und müssen in hohen Dosierungen eingesetzt werden.

An neue Duftstoffe werden sehr hohe Anforderungen gestellt. Sie sollten eine sehr gute biologische Abbaubarkeit aufweisen und dermatologisch sowie toxikologisch unbedenklich sein.

Der Geruch "ambra" oder auch "amber" stellt eine der hochbevorzugten Geruchsnoten in Parfüms dar. Er verleiht Parfüms eine besondere Exklusivität und Luxuriosität. Ursprünglich wurde Amber aus dem Verdauungstrakt von Pottwalen gewonnen, heutzutage werden Ambra-Düfte aus anderen Quellen bevorzugt. Nachteilig an vielen Ambra-Duftstoffen, ist, dass ihr Geruchseindruck erst allmählich wahrgenommen wird, weshalb es schwer ist, den Ambrageruch in der Topnote eines Parfüms darzustellen.

Holzige Geruchsnoten werden sehr oft in Parfüms verwendet und zählen zu den sehr beliebten Geruchsnoten. Ambranoten, die zudem auch holzig riechen werden als besonders hochwertig empfunden.

### Aufgabe der Erfindung

Die Aufgabe der Erfindung bestand darin, neue Verbindungen zur Verfügung zu stellen, welche die eingangs genannten Nachteile des Standes der Technik nicht aufweisen oder abmildern. Insbesondere bestand die Aufgabe der Erfindung darin, Duftstoffe zur Verfügung zu stellen, welche die Geruchsnote "ambra" und / oder holzig darstellen können und darüber hinaus über positive sekundäre Eigenschaften verfügen.

### Lösung der Aufgabe

Die Aufgabe wird gelöst durch Verbindungen der Formel I: 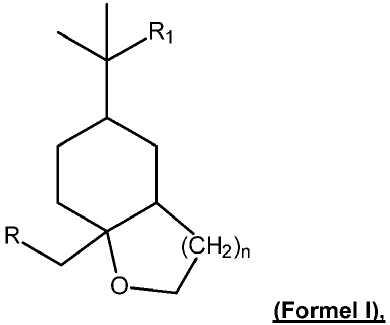 wobei die variablen Reste die folgende Bedeutung haben:
R = -H, -methyl, ethyl, O-methyl oder O-ethyl,
R₁₌-H, -methyl, ethyl, O-methyl oder O-ethyl und
n = 1 oder 2.

Die erfindungsgemäßen Stoffe können als 5,7a-Dialkyl-octahydro-benzofurane bzw. 6,8a-Dialkyl-octahydro-chromene bezeichnet werden.

Überraschend weisen die erfindungsgemäßen Verbindungen eine ambra-und/oder holzige Geruchsnote auf. Darüber hinaus weisen sie eine hohe Stabilität und Ausgiebigkeit, ein sehr gutes Haftungsvermögen, eine sehr große Ausstrahlungskraft, einen niedrigen Geruchsschwellenwert, eine sehr gute Löslichkeit und Mischbarkeit, eine geringe Neigung zu Reaktionen mit anderen Duftstoffen, eine sehr gute dermatologische und toxikologische Verträglichkeit sowie eine sehr gute biologische Abbaubarkeit auf.

Ein weiterer Vorteil der erfindungsgemäßen Verbindungen ist ihre hohe Duftintensität bei vergleichsweise geringer Dosierung. Dies ist aus Gründen der Umweltreinhaltung von besonderem Interesse, da hierdurch die Menge der in die Umwelt freigesetzten Stoffe minimal gehalten werden kann. Weiterhin wird die Ambranote besonders schnell wahrgenommen, so dass sich die Verbindungen beispielsweise als Topnote in einem Parfümöl eignen.

Die Verbindungen der Formel I selbst sind sehr stabil und weisen ebenso eine hohe Stabilität unter ungünstigen Bedingungen, wie hohem Sauerstoffgehalt, hohem Gehalt an Oxidationsmitteln oder Reduktionsmitteln, hohen Temperaturen und unter extremen pH-Bedingungen auf.

Die erfindungsgemäßen Verbindungen können als Racemate oder einzelne Enantiomere vorliegen.

Bevorzugt beträgt das Molgewicht der erfindungsgemäßen Verbindungen mindestens 182 und höchstens 240 g/mol.

Weiter bevorzugte Verbindungen sind Verbindungen der Formel I, wobei die variablen Reste die folgende Bedeutung haben:
R = -H, -methyl, -ethyl, O-methyl oder O-ethyl,
R₁₌-H, -methyl, -ethyl, O-methyl oder O-ethyl und
n = 1.

Insbesondere bevorzugt sind diese konkreten Verbindungen:

**Tabelle 1: Konkrete erfindungsgemäße Verbindungen sowie deren Geruchsbeschreibungen**

| **Verbindung** | **Molgewicht** | **Struktur** | **Geruch** |
|---|---|---|---|
| **1** | **182** | 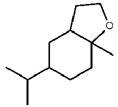 | strahlend, ambra, holzig |
| **2** | **196** | 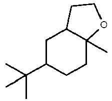 | stark, ambra, holzig |
| **3** | **212** | 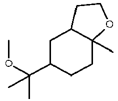 | strahlend, ambra, holzig |
| **4** | **210** | 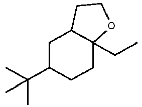 | ambra, holzig |
| **5** | **210** | 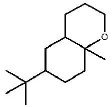 | ambra, holzig, animalisch |
| **6** | **196** | 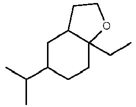 | strahlend, ambra, holzig |
| **7** | **210** | 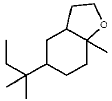 | ambra, holzig, animalisch |
| **8** | **196** | 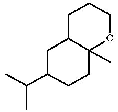 | ambra, holzig |

Die Verbindungen zeichnen sich dadurch aus, dass sie bei geringer Dosierung einen besonders komplexen, strahlenden und hochintensiven Ambraduft aufweisen. Darüber hinaus weisen sie einen holzigen Duft auf, so dass die besonders erwünschte Kombination aus Ambra- und Holznoten entsteht. Die Erfindung betrifft die Verbindungen als solche, einzeln oder auch Mischungen der erfindungsgemäßen Verbindungen.

Ein weiterer Gegenstand der Erfindung betrifft Mischungen enthaltend oder bestehend aus den erfindungsgemäßen Verbindungen (Komponenten a) und Lösungsmitteln (Komponenten b). Die erfindungsgemäßen Verbindungen sind gut in den verschiedensten Lösungsmitteln löslich. Bevorzugte Lösungsmittel, welche die erfindungsgemäßen Verbindungen besonders gut zu lösen vermögen sind ausgewählt aus der Gruppe, die gebildet wird von Ethanol, Isopropanol, Diethylenglycolmonoethylether, Glycerin, Propylenglycol, 1,2-Butylenglycol, Dipropylenglycol, Diethylphthalat, Triethylcitrat, Isopropylmyristat sowie deren Mischungen. Der Anteil der erfindungsgemäßen Verbindungen (Komponenten a) in diesen Mischungen beträgt bevorzugt 0,1 bis 50 Gew.-%, weiter bevorzugt 0,5-15 Gew.-% und besonders bevorzugt 0,75 bis 2 Gew.-%, bezogen auf das Gesamtgewicht der erfindungsgemäßen Verbindungen (Komponenten a) und der Lösungsmittel (Komponenten b).

Außerdem betrifft die Erfindung Mischungen enthaltend oder bestehend aus den erfindungsgemäßen Verbindungen (Komponenten a) und einem oder mehreren Duftstoff(en) (Komponenten c). Die erfindungsgemäßen Verbindungen sind besonders gut und in einem sehr weiten Konzentrationsverhältnis in anderen Duftstoffen löslich und weisen eine geringe Reaktivität mit anderen Duftstoffen auf. Die erfindungsgemäßen Verbindungen wirken in diesen Mischungen abrundend, harmonisierend und verbessern den natürlichen Geruchseindruck. Das Verhältnis aus der Gesamtmasse der erfindungsgemäßen Verbindungen (Komponenten a) zur Gesamtmasse der anderen Duftstoffe (Komponenten c) beträgt bevorzugt von 1:999 bis 999:1, weiter bevorzugt von 1:99 bis 1:1, insbesondere bevorzugt von 1:99 bis 1:2, weiter insbesondere bevorzugt 1:90 bis 1:5 und besonders bevorzugt von 1:80 bis 1:10.

Beispiele für Duftstoffe, mit denen die erfindungsgemäßen Verbindungen vorteilhaft kombiniert werden können, finden sich z. B. in "S. Arctander, Perfume and Flavor Chemicals, Vol. I und II, Montclair, N. J., 1969, Selbstverlag" oder "H. Surburg und J. Panten, Common Fragrance and Flavor Materials, 5th ed., Wiley-VCH, Weinheim, 2006". Im Einzelnen seien genannt:
**Extrakte aus natürlichen Rohstoffen** wie Etherische Öle, Concretes, Absolues, Resine, Resinoide, Balsame, Tinkturen wie z. B.

Ambratinktur; Amyrisöl; Angelicasamenöl; Angelicawurzelöl; Anisöl; Baldrianöl; Basilikumöl; Baummoos -Absolue; Bayöl; Beifußöl; Benzoeresin; Bergamotteöl; Bienenwachs-Absolue; Birkenteeröl; Bittermandelöl; Bohnenkrautöl; Buccoblätteröl; Cabreuvaöl; Cadeöl; Calmusöl; Campheröl; Canangaöl; Cardamomenöl; Cascarillaöl; Cassiaöl; Cassie-Absolue; Castoreumabsolue; Cedernblätteröl; Cedernholzöl; Cistusöl; Citronellöl; Citronenöl; Copaivabalsam; Copaivabalsamöl; Corianderöl; Costuswurzelöl; Cuminöl; Cypressenöl; Davanaöl; Dillkrautöl; Dillsamenöl; Eau de brouts-Absolue; Eichenmoos-Absolue; Elemiöl; Estragonöl; Eucalyptuscitriodora-ÖI; Eucalyptusöl; Fenchelöl ; Fichtennadelöl; Galbanumöl; Galbanumresin; Geraniumöl; Grapefruitöl; Guajakholzöl; Gurjunbalsam; Gurjunbalsamöl; Helichrysum-Absolue; Helichrysumöl; Ingweröl; Iriswurzel-Absolue; Iriswurzelöl; Jasmin-Absolue; Kalmusöl; Kamillenöl blau; Kamillenöl römisch; Karottensamenöl; Kaskarillaöl; Kiefernadelöl; Krauseminzöl; Kümmelöl; Labdanumöl; Labdanum-Absolue; Labdanumresin; Lavandin-Absolue; Lavandinöl ; Lavendel-Absolue; Lavendelöl; Lemongrasöl; Liebstocköl; Limetteöl destilliert; Limetteöl gepreßt; Linaloeöl; Litsea-cubeba-Öl; Lorbeerblätteröl; Macisöl; Majoranöl; Mandarinenöl; Massoirindenöl; Mimosa-Absolue; Moschuskörneröl; Moschustinktur; Muskateller-Salbei-Öl; Muskatnußöl; Myrrhen-Absolue; Myrrhenöl; Myrtenöl; Nelkenblätteröl; Nelkenblütenöl; Neroliöl; Olibanum-Absolue; Olibanumöl; Opopanaxöl; Orangenblüten-Absolue; Orangenöl; Origanumöl; Palmarosaöl; Patchouliöl; Perillaöl; Perubalsamöl; Petersilienblätteröl; Petersiliensamenöl; Petitgrainöl; Pfefferminzöl; Pfefferöl; Pimentöl; Pineöl; Poleyöl; Rosen-Absolue; Rosenholzöl; Rosenöl; Rosmarinöl; Salbeiöl dalmatinisch; Salbeiöl spanisch; Sandelholzöl; Selleriesamenöl; Spiklavendelöl; Sternanisöl; Styraxöl; Tagetesöl; Tannennadelöl; Tea-tree-Öl; Terpentinöl; Thymianöl; Tolubalsam; Tonka-Absolue; Tuberosen-Absolue; Vanilleextrakt; Veilchenblätter-Absolue; Verbenaöl; Vetiveröl; Wacholderbeeröl; Weinhefenöl; Wermutöl; Wintergrünöl; Ylangöl; Ysopöl; Zibet-Absolue; Zimtblätteröl; Zimtrindenöl sowie Fraktionen davon, bzw. daraus isolierten Inhaltsstoffen;

**Einzel-Riechstoffe** aus der Gruppe der Kohlenwasserstoffe, wie z.B. 3-Caren; α-Pinen; β-Pinen; α-Terpinen; γ-Terpinen; p-Cymol; Bisabolen; Camphen; Caryophyllen; Cedren; Farnesen; Limonen; Longifolen; Myrcen; Ocimen; Valencen; (E,Z)-1,3,5-Undecatrien; Styrol; Diphenylmethan;
der aliphatischen Alkohole wie z.B. Hexanol; Octanol; 3-Octanol; 2,6-Dimethylheptanol; 2-Methyl-2-heptanol; 2-Methyl-2-octanol; (E)-2-Hexenol; (E)- und (Z)-3-Hexenol; 1-Octen-3-ol; Gemisch von 3,4,5,6,6-Pentamethyl-3/4-hepten-2-ol und 3,5,6,6-Tetramethyl-4-methyleneheptan-2-ol; (E,Z)-2,6-Nonadienol; 3,7-Dimethyl-7-methoxyoctan-2-ol; 9-Decenol; 10-Undecenol; 4-Methyl-3-decen-5-ol;
der aliphatischen Aldehyde und deren Acetale wie z.B. Hexanal; Heptanal; Octanal; Nonanal; Decanal; Undecanal; Dodecanal; Tridecanal; 2-Methyloctanal; 2-Methylnonanal; (E)-2-Hexenal; (Z)-4-Heptenal; 2,6-Dimethyl-5-heptenal; 10-Undecenal; (E)-4-Decenal; 2-Dodecenal;2,6,10-Trimethyl-9-undecenal; 2,6,10-Trimethyl-5,9-undecadienal; Heptanaldiethylacetal; 1,1-Dimethoxy-2,2,5-trimethyl-4-hexen; Citronellyloxyacetaldehyd; 1-(1-Methoxy-propoxy)-(E/Z)-3-hexen;
der aliphatischen Ketone und deren Oxime wie z.B. 2-Heptanon; 2-Octanon; 3-Octanon; 2-Nonanon; 5-Methyl-3-heptanon ; 5-Methyl-3-heptanonoxim; 2,4,4,7-Tetramethyl-6-octen-3-on; 6-Methyl-5-hepten-2-on;
der aliphatischen schwefelhaltigen Verbindungen wie z.B. 3-Methylthio-hexanol; 3-Methylthiohexylacetat; 3-Mercaptohexanol; 3-Mercaptohexylacetat; 3-Mercaptohexylbutyrat; 3-Acetylthiohexylacetat; 1-Menthen-8-thiol;
der aliphatischen Nitrile wie z.B. 2-Nonensäurenitril; 2-Undecensäurenitril; 2-Tridecensäurenitril; 3,12-Tridecadiensäurenitril; 3,7-Dimethyl-2,6-octadien-säurenitril; 3,7-Dimethyl-6-octensäurenitril;
der Ester von aliphatischen Carbonsäuren wie z.B. (E)- und (Z)-3-Hexenylformiat; Ethylacetoacetat; Isoamylacetat; Hexylacetat; 3,5,5-Trimethylhexylacetat; 3-Methyl-2-butenylacetat; (E)-2-Hexenylacetat; (E)- und (Z)-3-Hexenylacetat; Octylacetat; 3-Octylacetat; 1-Octen-3-ylacetat; Ethylbutyrat; Butylbutyrat, ; Isoamylbutyrat; Hexylbutyrat; (E)- und (Z)-3-Hexenyl-isobutyrat; Hexylcrotonat; Ethylisovalerianat; Ethyl-2-methylpentanoat; Ethylhexanoat; Allylhexanoat; Ethylheptanoat; Allylheptanoat; Ethyloctanoat; Ethyl-(E,Z)-2,4-decadienoat; Methyl-2-octinat; Methyl-2-noninat; Allyl-2-isoamyloxyacetat; Methyl-3,7-dimethyl-2,6-octadienoat;4-Methyl-2-pentyl-crotonat;
der acyclischen Terpenalkohole wie z. B. Citronellol; Geraniol; Nerol; Linalool; Lavadulol; Nerolidol; Farnesol; Tetrahydrolinalool; Tetrahydrogeraniol; 2,6-Dimethyl-7-octen-2-ol; 2,6-Dimethyloctan-2-ol; 2-Methyl-6-methylen-7-octen-2-ol; 2,6-Dimethyl-5,7-octadien-2-ol; 2,6-Dimethyl-3,5-octadien-2-ol; 3,7-Dimethyl-4,6-octadien-3-ol; 3,7-Dimethyl-1,5,7-octatrien-3-ol 2,6-Dimethyl-2,5,7-octatrien-1-ol; sowie deren Formiate, Acetate, Propionate, Isobutyrate, Butyrate, Isovalerianate, Pentanoate, Hexanoate, Crotonate, Tiglinate und 3-Methyl-2-butenoate;
der acyclischen Terpenaldehyde und -ketone wie z. B. Geranial; Neral; Citronellal; 7-Hydroxy-3,7-dimethyloctanal; 7-Methoxy-3,7-dimethyloctanal; 2,6,10-Trimethyl-9-undecenal; Geranylaceton; sowie die Dimethyl- und Diethylacetale von Geranial, Neral, 7-Hydroxy-3,7-dimethyloctanal;
der cyclischen Terpenalkohole wie z. B. Menthol; Isopulegol; alpha-Terpineol; Terpinenol-4; Menthan-8-ol; Menthan-1-ol; Menthan-7-ol; Borneol; Isoborneol; Linalooloxid; Nopol; Cedrol; Ambrinol; Vetiverol; Guajol; sowie deren Formiate, Acetate, Propionate, Isobutyrate, Butyrate, Isovalerianate, Pentanoate, Hexanoate, Crotonate, Tiglinate und 3-Methyl-2-butenoate;
der cyclischen Terpenaldehyde und -ketone wie z. B. Menthon; Isomenthon ; 8-Mercaptomenthan-3-on ; Carvon; Campher; Fenchon; alpha-Ionon; beta-Ionon; alpha-n-Methylionon; beta-n-Methylionon; alpha-Isomethylionon; beta-Isomethylionon; alpha-Iron; alpha-Damascon; beta-Damascon; beta-Damascenon; delta-Damascon; gamma-Damascon; 1-(2,4,4-Trimethyl-2-cyclohexen-1-yl)-2-buten-1-on; 1,3,4,6,7,8a-Hexahydro-1,1,5,5-tetramethyl-2H-2,4a-methanonaphthalen-8(5H)-on;2-Methyl-4-(2,6,6-trimethyl-1-cyclohexen-1-yl)-2-butenal; Nootkaton ; Dihydronootkaton ; 4,6,8-Megastigmatrien-3-on; alpha-Sinensal ; beta-Sinensal ; acetyliertes Cedernholzöl (Methylcedrylketon);
der cyclischen Alkohole wie z.B. 4-tert.-Butylcyclohexanol ; 3,3,5-Trimethylcyclohexanol; 3-Isocamphylcyclohexanol; 2,6,9-Trimethyl-Z2,Z5,E9-cyclododecatrien-1-ol; 2-Isobutyl-4-methyltetrahydro-2H-pyran-4-ol;
der cycloaliphatischen Alkohole wie z.B. alpha,3,3-Trimethylcyclohexylmethanol;1-(4-Isopropylcyclohexyl)ethanol; 2-Methyl-4-(2,2,3-trimethyl-3-cyclopent-1-yl)butanol; 2-Methyl-4-(2,2,3-trimethyl-3-cyclopent-1-yl)-2-buten-1-ol; 2-Ethyl-4-(2,2,3-trimethyl-3-cyclopent-1-yl)-2-buten-1-ol; 3-Methyl-5-(2,2,3-trimethyl-3-cyclopent-1-yl)-pentan-2-ol; 3-Methyl-5-(2,2,3-trimethyl-3-cyclopent-1-yl)-4-penten-2-ol; 3,3-Dimethyl-5-(2,2,3-trimethyl-3-cyclopent-1-yl)-4-penten-2-ol; 1-(2,2,6-Trimethylcyclohexyl)pentan-3-ol; 1-(2,2,6-Trimethylcyclohexyl)hexan-3-ol;
der cyclischen und cycloaliphatischen Ether wie z.B. Cineol; Cedrylmethylether; Cyclododecylmethylether;1,1-Dimethoxycyclododecan; (Ethoxymethoxy)cyclododecan; alpha-Cedrenepoxid; 3a,6,6,9a-Tetramethyldodecahydronaphtho[2,1-b]furan; 3a-Ethyl-6,6,9a-trimethyldodecahydronaphtho[2,1-b]furan; 1,5,9-Trimethyl-13-oxabicyclo[10.1.0]trideca-4,8-dien; Rosenoxid; 2-(2,4-Dimethyl-3-cyclohexen-1-yl)-5-methyl-5-(1-methylpropyl)-1,3-dioxan;
der cyclischen und makrocyclischen Ketone wie z.B. 4-tert.-Butylcyclohexanon; 2,2,5-Trimethyl-5-pentylcyclopentanon; 2-Heptylcyclopentanon; 2-Pentylcyclopentanon; 2-Hydroxy-3-methyl-2-cyclopenten-1-on; 3-Methyl-cis-2-penten-1-yl-2-cyclopenten-1-on; 3-Methyl-2-pentyl-2-cyclopenten-1-on; 3-Methyl-4-cyclopentadecenon; 3-Methyl-5-cyclopentadecenon; 3-Methylcyclopentadecanon; 4-(1-Ethoxyvinyl)-3,3,5,5-tetramethylcyclohexanon; 4-tert.-Pentylcyclohexanon; 5-Cyclohexadecen-1-on; 6,7-Dihydro-1,1,2,3,3-pentamethyl-4(5H)-indanon; 8-Cyclohexadecen-1-on; 9-Cycloheptadecen-1-on; Cyclopentadecanon; Cyclohexadecanon;
der cycloaliphatischen Aldehyde wie z.B. 2,4-Dimethyl-3-cyclohexencarbaldehyd; 2-Methyl-4-(2,2,6-trimethyl-cyclohexen-1-yl)-2-butenal; 4-(4-Hydroxy-4-methylpentyl)-3-cyclohexencarbaldehyd; 4-(4-Methyl-3-penten-1-yl)-3-cyclohexencarbaldehyd;
der cycloaliphatischen Ketone wie z. B. 1-(3,3-Dimethylcyclohexyl)-4-penten-1-on; 2,2-Dimethyl-1-(2,4-dimethyl-3-cyclohexen-1-yl)-1-propanon; 1-(5,5-Dimethyl-1-cyclohexen-1-yl)-4-penten-1-on; 2,3,8,8-Tetramethyl-1,2,3,4,5,6,7,8-octahydro-2-naphtalenylmethylketon; Methyl-2,6,10-trimethyl-2,5,9-cyclododecatrienylketon; tert.-Butyl-(2,4-dimethyl-3-cyclohexen-1-yl)keton;
der Ester cyclischer Alkohole wie z.B. 2-tert-Butylcyclohexylacetat; 4-tert-Butylcyclohexylacetat; 2-tert-Pentylcyclohexylacetat; 4-tert-Pentylcyclohexylacetat; 3,3,5-Trimethylcyclohexylacetat; Decahydro-2-naphthylacetat;2-Cyclopentylcyclopentylcrotonat; 3-Pentyltetrahydro-2H-pyran-4-ylacetat; Decahydro-2,5,5,8a-tetramethyl-2-naphthylacetat; 4,7-Methano-3a,4,5,6,7,7a-hexahydro-5, bzw. 6-indenylacetat; 4,7-Methano-3a,4,5,6,7,7a-hexahydro-5, bzw. 6-indenylpropionat; 4,7-Methano-3a,4,5,6,7,7a-hexahydro-5, bzw. 6-indenylisobutyrat; 4,7-Methanooctahydro-5, bzw. 6-indenylacetat;
der Ester cycloaliphatischer Alkohole wie z.B.1-Cyclohexylethylcrotonat;
der Ester cycloaliphatischer Carbonsäuren wie z. B. Allyl-3-cyclohexylpropionat; Allylcyclohexyloxyacetat; cis- und trans-Methyldihydrojasmonat; cis- und trans-Methyljasmonat; Methyl-2-hexyl-3-oxocyclopentancarboxylat; Ethyl-2-ethyl-6,6-dimethyl-2-cyclohexencarboxylat; Ethyl-2,3,6,6-tetramethyl-2-cyclohexencarboxylat; Ethyl-2-methyl-1,3-dioxolan-2-acetat;
der araliphatischen Alkohole wie z.B. Benzylalkohol; 1-Phenylethylalkohol; 2-Phenylethylalkohol; 3-Phenylpropanol; 2-Phenylpropanol; 2-Phenoxyethanol; 2,2-Dimethyl-3-phenylpropanol; 2,2-Dimethyl-3-(3-methylphenyl)propanol; 1,1-Dimethyl-2-phenylethylalkohol; 1,1-Dimethyl-3-phenylpropanol; 1-Ethyl-1-methyl-3-phenylpropanol; 2-Methyl-5-phenylpentanol; 3-Methyl-5-phenylpentanol; 3-Phenyl-2-propen-1-ol; 4-Methoxybenzylalkohol; 1-(4-Isopropylphenyl)ethanol;
der Ester von araliphatischen Alkoholen und aliphatischen Carbonsäuren wie z.B. Benzylacetat; Benzylpropionat; Benzylisobutyrat; Benzylisovalerianat; 2-Phenylethylacetat; 2-Phenylethylpropionat; 2-Phenylethylisobutyrat; 2-Phenylethylisovalerianat; 1-Phenylethylacetat; alpha-Trichlormethylbenzylacetat; alpha,alpha-Dimethylphenylethylacetat; alpha,alpha-Dimethylphenylethylbutyrat; Cinnamylacetat; 2-Phenoxyethylisobutyrat; 4-Methoxybenzylacetat;
der araliphatischen Ether wie z.B. 2-Phenylethylmethylether; 2-Phenylethylisoamylether; 2-Phenylethyl-1-ethoxyethylether; Phenylacetaldehyddimethylacetal; Phenylacetaldehyddiethylacetal; Hydratropaaldehyddimethylacetal; Phenylacetaldehydglycerinacetal; 2,4,6-Trimethyl-4-phenyl-1,3-dioxan; 4,4a,5,9b-Tetrahydroindeno[1,2-d]-m-dioxin; 4,4a,5,9b-Tetrahydro-2,4-dimethylindeno[1,2-d]-m-dioxin;
der aromatischen und araliphatischen Aldehyde wie z. B. Benzaldehyd; Phenylacetaldehyd; 3-Phenylpropanal; Hydratropaaldehyd; 4-Methylbenzaldehyd; 4-Methylphenylacetaldehyd; 3-(4-Ethylphenyl)-2,2-dimethylpropanal; 2-Methyl-3-(4-isopropylphenyl)propanal; 2-Methyl-3-(4-tert.-butylphenyl)propanal; 2-Methyl-3-(4-isobutylphenyl)propanal; 3-(4-tert.-Butylphenyl)propanal; Zimtaldehyd; alpha-Butylzimtaldehyd; alpha-Amylzimtaldehyd; alpha-Hexylzimtaldehyd; 3-Methyl-5-phenylpentanal; 4-Methoxybenzaldehyd; 4-Hydroxy-3-methoxybenzaldehyd; 4-Hydroxy-3-ethoxybenzaldehyd; 3,4-Methylendioxybenzaldehyd; 3,4-Dimethoxybenzaldehyd; 2-Methyl-3-(4-methoxyphenyl)propanal; 2-Methyl-3-(4-methylendioxyphenyl)propanal;
der aromatischen und araliphatischen Ketone wie z.B. Acetophenon; 4-Methylacetophenon; 4-Methoxyacetophenon; 4-tert.-Butyl-2,6-dimethylacetophenon; 4-Phenyl-2-butanon; 4-(4-Hydroxyphenyl)-2-butanon; 1-(2-Naphthalenyl)ethanon;2-Benzofuranylethanon;(3-Methyl-2-benzofuranyl)ethanon; Benzophenon; 1,1,2,3,3,6-Hexamethyl-5-indanylmethylketon; 6-tert.-Butyl-1,1-dimethyl-4-indanylmethylketon; 1-[2,3-dihydro-1,1,2,6-tetramethyl-3-(1-methylethyl)-1 H-5-indenyl]ethanon; 5',6',7',8'-Tetrahydro-3',5',5',6',8',8'-hexamethyl-2-acetonaphthon;
der aromatischen und araliphatischen Carbonsäuren und deren Ester wie z.B. Benzoesäure; Phenylessigsäure; Methylbenzoat; Ethylbenzoat; Hexylbenzoat; Benzyl-benzoat; Methylphenylacetat; Ethylphenylacetat; Geranylphenylacetat; Phenylethyl-phenylacetat; Methylcinnmat; Ethylcinnamat; Benzylcinnamat; Phenylethylcinnamat; Cinnamylcinnamat; Allylphenoxyacetat; Methylsalicylat; Isoamylsalicylat; Hexylsalicylat; Cyclohexylsalicylat; Cis-3-Hexenylsalicylat; Benzylsalicylat; Phenylethylsalicylat; Methyl-2,4-dihydroxy-3,6-dimethylbenzoat; Ethyl-3-phenylglycidat; Ethyl-3-methyl-3-phenylglycidat;
der stickstoffhaltigen aromatischen Verbindungen wie z.B. 2,4,6-Trinitro-1,3-dimethyl-5-tert.-butylbenzol; 3,5-Dinitro-2,6-dimethyl-4-tert.-butylacetophenon; Zimtsäurenitril; 3-Methyl-5-phenyl-2-pentensäurenitril; 3-Methyl-5-phenylpentansäurenitril; Methylanthranilat; Methy-N-methylanthranilat; Schiff'sche Basen von Methylanthranilat mit 7-Hydroxy-3,7-dimethyloctanal, 2-Methyl-3-(4-tert.-butylphenyl)propanal oder 2,4-Dimethyl-3-cyclohexencarbaldehyd; 6-Isopropylchinolin; 6-Isobutylchinolin; 6-sec.-Butylchinolin;2-(3-Phenylpropyl)pyridin; Indol; Skatol; 2-Methoxy-3-isopropylpyrazin; 2-Isobutyl-3-methoxypyrazin;
der Phenole, Phenylether und Phenylester wie z.B. Estragol; Anethol; Eugenol; Eugenylmethylether; Isoeugenol; Isoeugenylmethylether; Thymol; Carvacrol; Diphenylether; beta-Naphthylmethylether; beta-Naphthylethylether; beta-Naphthylisobutylether; 1,4-Dimethoxybenzol; Eugenylacetat; 2-Methoxy-4-methylphenol; 2-Ethoxy-5-(1-propenyl)phenol; p-Kresylphenylacetat;
der heterocyclischen Verbindungen wie z.B. 2,5-Dimethyl-4-hydroxy-2H-furan-3-on; 2-Ethyl-4-hydroxy-5-methyl-2H-furan-3-on; 3-Hydroxy-2-methyl-4H-pyran-4-on; 2-Ethyl-3-hydroxy-4H-pyran-4-on;
der Lactone wie z.B. 1,4-Octanolid; 3-Methyl-1,4-octanolid; 1,4-Nonanolid; 1,4-Decanolid; 8-Decen-1,4-olid; 1,4-Undecanolid; 1,4-Dodecanolid; 1,5-Decanolid; 1,5-Dodecanolid;4-Methyl-1,4-decanolid; 1,15-Pentadecanolid; cis- und trans-11-Pentadecen-1,15-olid; cis- und trans-12-Pentadecen-1,15-olid; 1,16-Hexadecanolid; 9-Hexadecen-1,16-olid; 10-Oxa-1,16-hexadecanolid; 11-Oxa-1,16-hexadecanolid; 12-Oxa-1,16-hexadecanolid; Ethylen-1,12-dodecandioat; Ethylen-1,13-tridecandioat; Cumarin; 2,3-Dihydrocumarin; Octahydrocumarin.

Die Erfindung betrifft ebenso Mischungen, die erfindungsgemäße Verbindungen (Komponenten a), ein oder mehrere Lösungsmittel (Komponenten b) sowie einen oder mehrere Duftstoffe (Komponenten c) enthalten oder aus diesen bestehen. Insbesondere handelt es sich bei solchen Mischungen um Parfümöle, Parfüms, Parfüm-Extraits, Eau de Parfums, Eau de Toilettes, Rasierwässer, Eau de Colognes, Pre-shave-Produkte, Splash-Colognes. Die erfindungsgemäßen Verbindungen bilden dabei insbesondere die Topnote der Parfüms oder der Parfümöle. Zur Topnote eines Parfümöls gehören diejenigen Noten, deren Geruch besonders schnell wahrgenommen werden kann. Bezogen auf die Gesamtmasse der Komponenten a, b und c beträgt der Massenanteil der Komponenten a und c zusammen bevorzugt 0,1 bis 50 Gew.-%, wobei das Massenverhältnis der Komponenten a zu c im Bereich 1:90 bis 1:5 liegt.

Ferner betrifft die Erfindung Konsumgüter einschließlich deren Vor- und Zwischenprodukte enthaltend die erfindungsgemäßen Verbindungen oder deren Mischungen mit Lösungsmitteln oder/ und weiteren Duftstoffen. Konsumgüter enthalten oft eine Vielzahl unterschiedlicher chemischer Verbindungen. Der Einsatz der erfindungsgemäßen Verbindungen ist insbesondere vorteilhaft wegen deren geringer Reaktivität, so dass weder die erfindungsgemäßen Verbindungen noch die Inhaltsstoffe der Konsumgüter nachteilig verändert werden. Zudem beeinflussen die erfindungsgemäßen Verbindungen in den zur Erzielung der erwünschten Dufwirkung notwendigen Konzentrationen nicht nachteilig die physikalischen oder physikochemischen Eigenschaften der Konsumgüter (z.B. die Viskosität oder den pH-Wert). Der Massenanteil der erfindungsgemäßen Verbindungen in den Konsumgütern beträgt bevorzugt 0,0001 bis 5 Gew.-%, weiter bevorzugt 0,001 bis 2 Gew.-% und besonders bevorzugt 0,001 bis 1 %.

Konsumgüter im Sinne der Erfindung sind insbesondere Kosmetika, Wasch- und Reinigungsmittel, Weichspüler, Bleichmittel, Desinfektionsmittel, Duftfreigabesysteme, Sonnenschutzmittel für die Haut, Haarpflegemittel und Deodorants. Besonders handelt es sich bei Konsumgütern um saure, alkalische und neutrale Reinigungsmittel, wie z.B. Fußbodenreiniger, Fensterglasreiniger, Geschirrspülmittel, Bad- und Sanitärreiniger, Scheuermilch, feste und flüssige WC-Reiniger, pulver- und schaumförmige Teppichreiniger, flüssige Waschmittel, pulverförmige Waschmittel, Wäschevorbehandlungsmittel wie Bleichmittel, Einweichmittel und Fleckenentferner, Wäscheweichspüler, Waschseifen, Waschtabletten, Desinfektionsmittel, Oberflächendes-infektionsmittel sowie Luftverbesserer in flüssiger, gelartiger oder auf einem festen Träger aufgebrachter Form einschließlich zur Beduftung von Toiletten, Aerosolsprays, Wachse und Polituren wie Möbelpolituren, Fußbodenwachse, Schuhcremes sowie Körperpflegemittel wie z.B. feste und flüssige Seifen, Duschgele, Shampoos, Rasierseifen, Rasierschäume, Badeöle, kosmetische Emulsionen vom Öl-in-Wasser-, vom Wasser-in-ÖI- und vom Wasser-in-Öl-in-Wasser-Typ wie z.B. Hautcremes- und -lotionen, Gesichtscremes und -lotionen, Sonnenschutzcremes- und -lotionen, After-sun-cremes und -lotionen, Handcremes und -lotionen, Fußcremes und -lotionen, Enthaarungscremes und -lotionen, After-Shaves, After Shave-Cremes und Lotionen, Pre-Shaves, Bräunungscremes und -lotionen, Haarpflegeprodukte wie z.B. Haarsprays, Haargele, Haarlotionen, Haarspülungen, permanente und semipermanente Haarfärbemittel, Haarverformungsmittel wie Kaltwellen und Haarglättungsmittel, Haarwässer, Haarcremes und -lotionen, Erfrischungstücher, Deodorantien und Antiperspirantien wie z.B. Achselsprays, Roll-ons, Deosticks, Deocremes oder Produkte der dekorativen Kosmetik wie zum Beispiel Make-Up.

Bei den in Konsumgütern enthaltenen Stoffen handelt es sich oftmals um Konservierungsmittel, Abrasiva, Antiakne-Mittel, Mittel gegen Hautalterung, anitbakterielle Mittel, Anticellulitis-Mittel, Antischuppen-Mittel, entzündungshemmende Mittel, irritationsverhindernde Mittel, irritationshemmende Mittel, antimikrobielle Mittel, Antioxidantien, Adstringentien, schweisshemmende Mittel, antiseptische Mittel, Antistatika, Binder, Puffer, Trägermaterialien, Chelatbildnder, Zellstimulantien, reinigende Mittel, pflegende Mittel, Enthaarungsmittel, oberflächenaktive Substanzen, deodorierende Mittel, Antiperspirantien, Weichmacher, Emulgatoren, Enzyme, ätherische Öle, Fasern, Filmbildner, Fixateure, Schaumbildner, Schaumstabilisatoren, Substanzen zum Verhindern des Schäumens, Schaumbooster, Fungizide, gelierende Mittel, gelbildende Mittel, Haarpflegemittel, Haarverformungsmittel, Haarglättungsmittel, feuchtigkeitsspendende Mittel, anfeuchtende Substanzen, feuchthaltende Substanzen, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, Pulver, Proteine, rückfettende Mittel, abschleifende Mittel, Slilcone, hautberuhigende Mittel, hautreinigende Mittel, hautpflegende Mittel, hautheilende Mittel, Hautaufhellungsmittel, hautschützende Mittel, hauterweichende Mittel, kühlende Mittel, hautkühlende Mittel, wärmende Mittel, hautwärmende Mittel, Stabilisatoren, UVabsorbierende Mittel, UV-Filter, Waschmittel, Weichspüler, suspendierende Mittel, Hautbräunungsmittel, Verdickungsmittel, Vitamine, Öle, Wachse, Fette, Phospholipide, gesättigte Fettsäuren, ein- oder mehrfach ungesättigte Fettsäuren, α-Hydroxysäuren, Polyhydroxyfettsäuren, Verflüssiger, Farbstoffe, farbschützende Mittel, Pigmente, Antikorrosiva, Aromen, Geschmackstoffe, Riechstoffe, Polyole, Tenside, Elektrolyte, organische Lösungsmittel oder Silikonderivate. Mit diesen Stoffen weisen die erfindungsgemäßen Verbindungen in Mischungen eine hohe Kompatibilität auf.

Die Erfindung betrifft auch die Verwendung der erfindungsgemäßen Stoffe. Die erfindungsgemäßen Stoffe können als Duftstoffe verwendet werden, insbesondere zur Erzeugung, zum Vermitteln, Modifizieren oder Verstärken eines ambrartigen und / oder holzigen Geruches. Besonders bevorzugt können die erfindungsgemäßen Verbindungen verwendet werden zur Erzeugung, zum Vermitteln, Modifizieren oder Verstärken einer ambrartigen und / oder holzigen Topnote eines Parfümöls. Außerdem lassen sich die erfindungsgemäßen Verbindungen zur Abrundung, Harmonisierung und zur Verbesserung der Natürlichkeit von Parfümölen und diesen enthaltenden Konsumprodukten einsetzen.

Neben dem Einsatz als Flüssigkeit in Lösungen oder in Emulsionen können die erfindungsgemäßen Verbindungen oder diese enthaltende Parfümöle an Feststoffen adsorbiert oder in Trägerstoffen (mikro)verkapselt eingesetzt werden. Diese Darreichungsformen können sowohl für eine feine Verteilung der Riechstoffe im Produkt als auch für eine kontrollierte Freisetzung bei der Anwendung sorgen. Derartige Feststoffe können poröse anorganische Materialien wie Leichtsulfat, Kieselgele, Zeolithe, Gipse, Tone, Tongranulate, Gasbeton usw. oder organische Materialien wie Hölzer, Cellulose-basierende Stoffe, Zucker oder Kunststoffe wie PVC, Polyvinylacetate oder Polyurethane sein. Verkapselungsprodukte können beispielsweise sprühgetrocknet, als Einschluß-Komplex oder als Extrusions-Produkt vorliegen. Die Mikroverkapselung der Parfümöle kann beispielsweise durch das sogenannte Koazervationsverfahren mit Hilfe von Kapselmaterialien z.B. aus polyurethanartigen Stoffen oder Weichgelatine erfolgen. Die sprühgetrockneten Parfümöle können beispielsweise durch Sprühtrocknung einer das Parfümöl enthaltenden Emulsion, bzw. Dispersion hergestellt werden, wobei als Trägerstoffe modifizierte Stärken, Proteine, Dextrin und pflanzliche Gummen verwendet werden können. Einschluß-Komplexe können z.B. durch Eintragen von Dispersionen von dem Parfümöl und Cyclodextrinen oder Harnstoffderivaten in ein geeignetes Lösungsmittel, z.B. Wasser, hergestellt werden. Extrusions-Produkte können durch Verschmelzen der Parfümöle mit einem geeigneten wachsartigen Stoff und durch Extrusion mit nachfolgender Erstarrung, ggf. in einem geeigneten Lösungsmittel, z.B. Isopropanol, erfolgen.

Die Adsorbate als auch die Verkapselungsprodukte können durch sogenanntes "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden können.

Die Erfindung betrifft auch ein Verfahren zur Herstellung der erfindungsgemäßen Verbindungen. Ausgehend von Verbindungen der Formel II 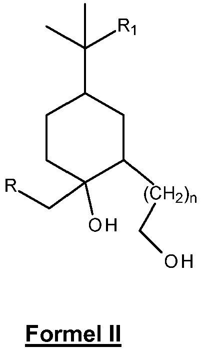 worin die variablen Reste
R = -H, -methyl, ethyl, O-methyl, O-ethyl
R₁ = -H, -methyl, ethyl, O-methyl, O-ethyl
n = 1,2

bedeuten, erhält man die erfindungsgemäßen Verbindungen durch intramolekulare Cyclisierung. R, R1 und n werden dabei analog zur herzustellenden erfindungsgemäßen Verbindung gewählt.

Die Ausgangsstoffe der Formel II sind entweder kommerziell erhältlich oder können durch Synthese gewonnen werden. Dies wird beispielhaft an den Verbindungen 2 und 5 demonstriert. Der Fachmann kann leicht die entsprechenden Reste variieren, um zu den anderen erfindungsgemäßen Verbindungen zu gelangen. Die Synthese kann z. B. ausgehend von tert.-Butyl-cyclohexanon in sechs Schritten durchgeführt werden:

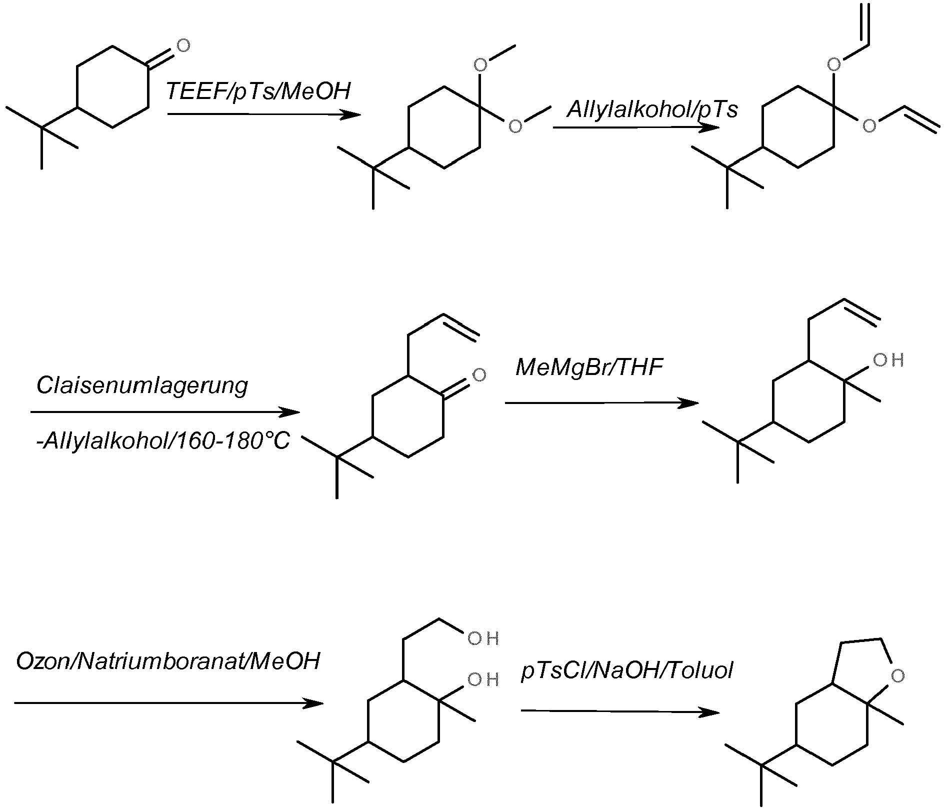

Die Synthese von Octahydrochromenen erfolgt z. B. anhand von 6-tert-butyl-8a-methyl-2,3,4,4a,5,6,7,8-octahydrochromene; methane, ausgehend vom Grignard-Produkt (siehe oben) in zwei Stufen:

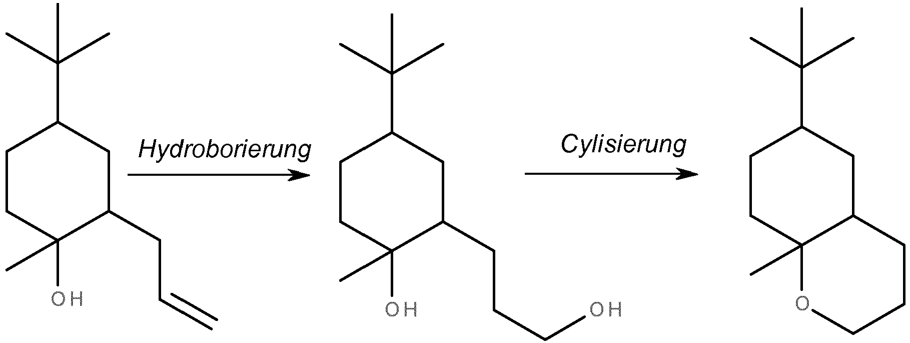

Alternativ kann die die Synthese des 5-tert-Butyl-7a-methyl-octahydro-benzofuran auch in vier Schritten erfolgen:

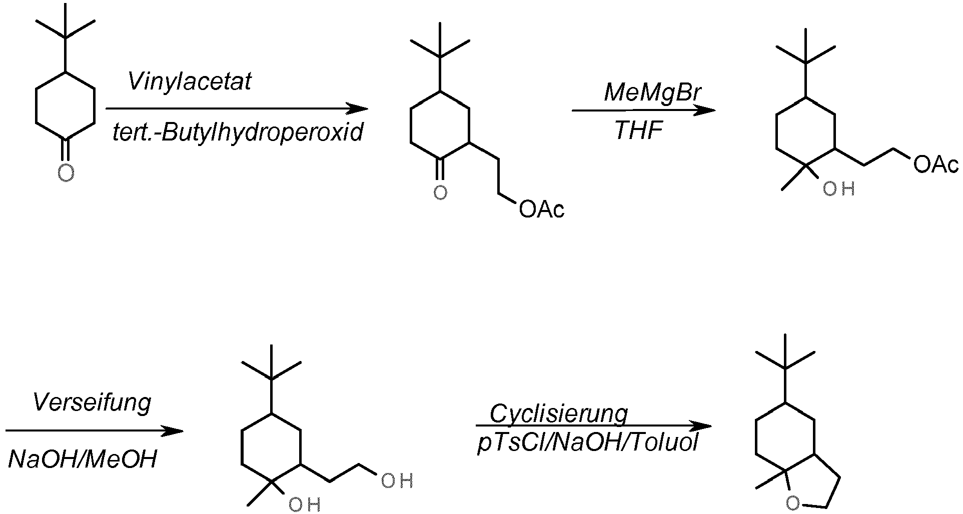

Als weitere Alternative kann die Synthese der Verbindungen über eine HWE-Reaktion mit Cyclsierung zum Lacton und anschließender Reduktion bzw. Cylisierung erfolgen:

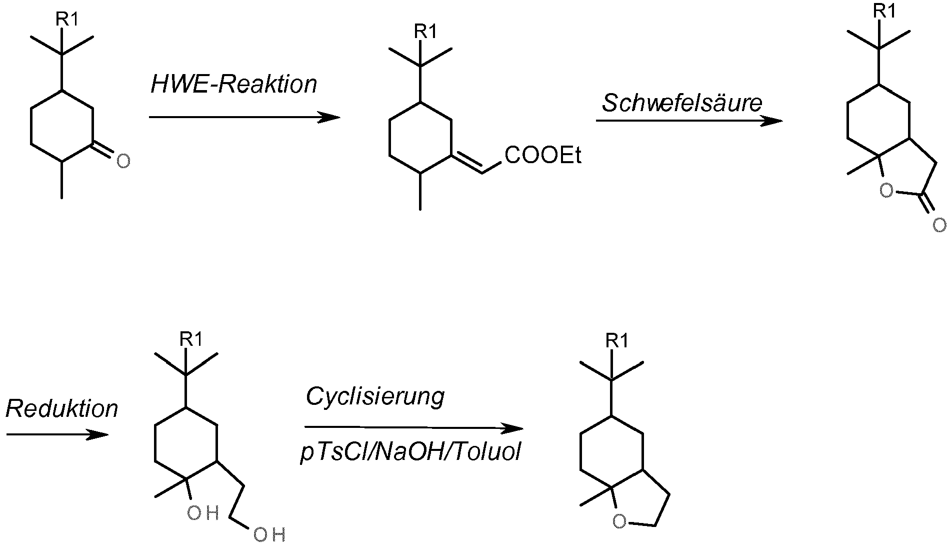

Es wurde festgestellt, dass die intramolekulare Cyclisierung der Verbindungen der Formel II zu Verbindungen der Formel I besonders gut, d.h. insbesondere mit hoher Ausbeute und in einem kurzen Zeitraum, in Gegenwart eines Sulfonsäurehalogenids (bevorzugt eines Sulfonsäurechlorids, von Toluolsulfonsäurechlorid, Benzolsulfonsäurechlorid und besonders bevorzugt von p- Toluolsulfonsäurechlorid) oder / und in Gegenwart von Tris(dioxa-3,6-heptyl)amin stattfindet. Weiterhin wirkt sich die Verwendung von Toluol als Lösungsmittel positiv auf die Reaktion aus. Ein weiterer Hilfsstoff für die Reaktion ist Natronlauge. Bevorzugte Temperaturen sind 30°C bis maximal 65°C und besonders bevorzugt 50-60°C.

### Beispiele

### Beispiel 1: Darstellung von 5-tert-Butyl-7a-methyl-octahydro-benzofuran

Darstellung des 4-tert.Butyl-cyclohexyl-diallylacetal

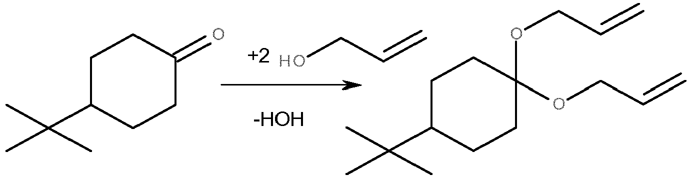

107,8 g (0,7 mol) 4-tert.Butylcyclohexanon, 79,8 g Acetondimethylacetal, 98 g Allylalkohol, 0,14 g p-Toluolsulfonsäure und 230 ml Cyclohexan wurden in einem 1 I Destillationskolben mit 40 cm Glasfüllkörperkolonne unter Normaldruck erhitzt, bis die Sumpftemperatur 91 °C betrug. Zur Aufarbeitung wurde mit einer Lösung von 0,15 g Natriummethylat in 14 ml Methanol versetzt, die organische Phase in Hexan aufgenommen, mit Wasser neutral neutral gewaschen und am Rotationsverdampfer eingeengt.
Rohausbeute: 174,3 g

Das Rohprodukt wird direkt in der folgenden Stufe eingesetzt.

Darstellung des 2-Allyl-4-tert.butyl-cyclohexanons

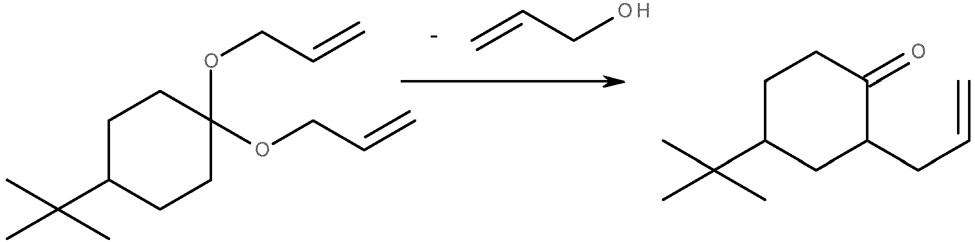

174,3 g Rohware aus der vorhergehenden Stufe, 0,15 g para-Toluolsulfonsäure und 200 ml Toluol wurden in einem 500 ml Destillationskolben mit 40 cm Glasfüllkörperkolonne bis zu einer Sumpftemperatur von 135°C erhitzt. Es gingen ca. 35 g Allylalkohol über. Zur Aufarbeitung wurde mit Sodalösung neutral gewaschen, die organische Phase mit Wasser gewaschen und am Rotationsverdampfer eingeengt.
Rohausbeute: 143,9 g

Anschließend wurde an einer 20cm-Vigreuxkolonne destilliert.
Ausbeute: 98,7g (Kp: 76°C/0,2mbar)

Darstellung von 1-Methyl-2-allyl-4-tert.butylcychexanol

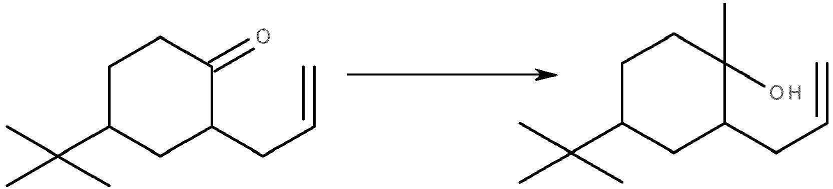

60 g Methylmagnesiumchlorid in 30 ml Tetrahydrofuran wasserfrei wurden unter Stickstoff in einem 250 ml Dreihalskolben mit Tropftrichter unter Wasserkühlung innerhalb von 1 h mit 29 g 2-Allyl-4-tert.butyl-cyclohexanons versetzt. Anschließend wurde 5 h unter Rückfluß erhitzt. Das Reaktionsgemisch wurde auf 80 g Eis gegeben und mit 3,5 ml Salzsäure angesäuert. Die organische Phase wurde anschließend mit Sodalösung und Wasser neutral gewaschen. Anschließend wurde am Rotationsverdampfer eingeengt und der Rückstand an einer Kugelrohrdestille destilliert.
Ausbeute: 20,1g

Die Substanz wurde ohne weitere Reinigung in die nächste Stufe eingesetzt.

Darstellung 4-tert. Butyl-2-(2-hydroxyethyl)-1-methyl-cyclohexanol

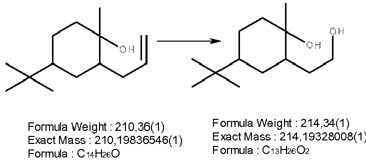

20,1 g (96 mmol) Rohprodukt aus der 3.Stufe wurden in 180 ml Methanol bei -5°C bis zum Ende der Ozonaufnahme ozonolisiert. Anschließend wurde mit einer Lösung aus 4,8 g Natriumborhydrid in 40 ml Natronlauge (1%) so versetzt, daß die Innentemperatur 0°C nicht überschritt. Die Lösung wurde mit Schwefelsäure (10%) angesäuert und anschließend mit 80ml Hexan extrahiert. Der entstandene Niederschlag wurde abfiltriert und die wäßrige Phase noch einmal mit 100ml MTB-Ether extrahiert. Die vereinigten organischen Phasen wurden neutral gewaschen, über Natriumsulfat getrocknet und anschließend am Rotationsverdampfer eingeengt.
Rohausbeute: 15,8 g

Die Rohware wurde über eine Kieselgelsäule (Länge: 50 cm, Durchmesser: 4 cm, 200 g Kieselgel, Laufmittel: Hexan/Essigester) gereinigt.
Ausbeute: 10,8 g

Darstellung von 5-tert.butyl-7a-methyl-3,3a-methyl-3,3a,4,5,6,7-hexahydro-2H-benzofuran

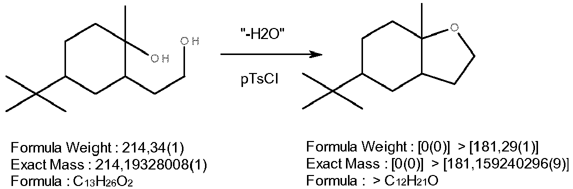

In einem 100 ml Dreihalskolben mit Magnetrührer, Kühler, Kontaktthermometer und Tropftrichter wurden 10,8 g Produkt aus Stufe 4, 14 ml Toluol, 0,05 g TDA-1 und 6,3 g Natronlauge (32%) vorgelegt und bei 60°C eine Lösung von 2,7 g p-Toluolsulphonsäurechlorid in 11 ml Toluol so zugetropft, dass die Innentemperatur nicht über 60°C stieg. Anschließend wurde 4 h bei 60°C gerührt. Zur Aufarbeitung wurde mit 1,9 g Natronlauge (32%) in 10 ml Ethanol versetzt und 2 h bei 60°C gerührt. Das Reaktionsgemisch wurde zweimal mit je 30 ml Wasser gewaschen, die organische Phase über Natriumsulfat getrocknet und das Lösungsmittel am Rotationsverdampfer abgezogen.
Rohausbeute: 6,9 g

Die Rohware wurde über eine Kieselgelsäule (Länge: 50 cm, Durchmesser: 4 cm, 250 g Kieselgel, Laufmittel: Hexan/Essigester) gereinigt.
Ausbeute: 2 g

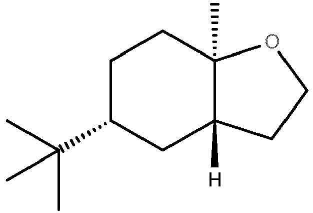

¹H-NMR (400 MHz, Chloroform-d): δ = 3.96-3.78 (m,2H), 2.11-1.93 (m,2H), 1.93-1.84 (m,1H), 1.84-1.76 (m,1H), 1.71-1.61 (m,1H), 1.59-1.42 (m,2H), 1.40-1.28 (m,1H), 1.27 (s,3H), 1.26-1.17 (m,1 H), 0.97 (tdd, J= 13.0, 11.7, 3.9 Hz, 1H), 0.86 (s, 9H).

¹³C-NMR (101 MHz, CDCl₃) δ = 80.32, 77.33, 77.02, 76.70, 64.70, 44.11, 41.48, 33.79, 32.11, 30.19, 27.59, 26.20, 24.81, 24.50.

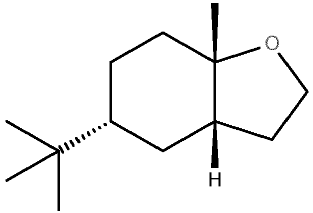

¹H-NMR (400 MHz, Chloroform-d): δ = 3.99-3.81 (m,2H), 2.36-2.20 (m,1H), 2.05-1.93 (m,1H), 1.77 (dt, *J=* 12.1, 6.3 Hz,1H), 1.63-1.46 (m,3H), 1.50-1.36 (m,1H), 1.25-1.11 (m,1H), 1.08 (s,3H), 1.02-0.89 (m,1 H), 0.84 (s, 9H), 0.89-0.77 (m, 1 H).

¹³C-NMR (101 MHz, CDCl₃) δ = 80.28, 77.34, 77.02, 76.70, 64.37, 46.81, 42.90, 35.95, 32.45, 32.32, 30.79, 27.47, 26.80, 22.74.

IR (Film): 715.7, 919.7, 1032.1, 1057.0, 1103.2, 1155.5, 1233.8, 1372.7, 1474.4, 1611.0, 1738.2, 28157, 2878.6, 2964.8, 3096.9 cm⁻¹.

MS (70 eV): 196, 181 (M-CH₃), 97, 57, 55, 43, 41.

Bei der Herstellung der anderen erfindungsgemäßen Verbindungen kann mit denselben Hilfsstoffen, Apparaturen und Parametern vorgegangen werden.

### Beispiele von Parfümölen:

### Parfümöl 1

| | | |
|---|---|---|
| AMBERWOOD® F | 7,000 | 7,000 |
| AMBRETTOLIDE | 6,000 | 6,000 |
| Verbindung 1 oder 2, 6, od. 8 | 0,000 | 34,000 |
| BHT IONOL | 3,000 | 3,000 |
| CASSIS BASE 345 BB | 10,000 | 10,000 |
| CITRONELLOL 950 | 10,000 | 10,000 |
| CITRONELLYL ACETATE EXTRA | 1,000 | 1,000 |
| DAMASCENONE 10% DPG | 5,000 | 5,000 |
| DAMASCONE ALPHA 10% DPG | 1,000 | 1,000 |
| DIPROPYLENE GLYCOL | 209,000 | 175,000 |
| ETHYLENE BRASSYLATE | 325,000 | 325,000 |
| GERANIOL SUPER | 20,000 | 20,000 |
| GERANYL ACETATE PURE | 1,000 | 1,000 |
| HEDIONE | 120,000 | 120,000 |
| HEDIONE HC/30 | 30,000 | 30,000 |
| HELIONAL | 4,000 | 4,000 |
| HEXENYL ACETATE CIS TRANS-3 10% DPG | 3,000 | 3,000 |
| HEXYL CINNAMIC ALDEHYDE ALPHA | 20,000 | 20,000 |
| HYDROXY CITRONELLAL | 15,000 | 15,000 |
| INDOLE FF 10% DPG | 1,000 | 1,000 |
| IONONE BETA | 8,000 | 8,000 |
| ISO E SUPER | 100,000 | 100,000 |
| LEMON OIL WINTER BERGAPTEN FREE | 10,000 | 10,000 |
| LINALOOL | 5,000 | 5,000 |
| LINALYL ACETATE | 25,000 | 25,000 |
| MAGNOLAN | 20,000 | 20,000 |
| MANDARIN OIL DIST. DECOL. | 5,000 | 5,000 |
| METHYL IONONE GAMMA PURE / IFF | 1,000 | 1,000 |
| METHYL OCTIN CARBONATE 1% DPG | 5,000 | 5,000 |
| NEROL 900 | 2,000 | 2,000 |
| PHENYLETHYL ALCOHOL | 20,000 | 20,000 |
| ROSESSENCE 193E | 3,000 | 3,000 |
| VERTOCITRAL 10% DPG | 5,000 | 5,000 |

mit Zusatz von Verbindung 1 od. 2, 6, od. 8 (siehe Liste) riecht die Mischung neben einer intensiveren Ambra- sowie Holznote harmonischer, blumiger und natürlicher.

### Parfümöl 2

| | | |
|---|---|---|
| AMBRETTOLIDE | 15,000 | 15,000 |
| **Verbindung 2, 3, 4, 5, oder 7** | 0,000 | 40,000 |
| BASIL OIL COMORES TYPE E 10% DPG | 2,000 | 2,000 |
| BERGAMOT OIL | 40,000 | 40,000 |
| BOURGEONAL 10% DPG | 5,000 | 5,000 |
| CARDAMOM OIL | 2,000 | 2,000 |
| CASHMERAN | 15,000 | 15,000 |
| CINNAMON BARK OIL CEYLON 10% DPG | 5,000 | 5,000 |
| CLARY SAGE OIL | 2,000 | 2,000 |
| CORIANDER OIL | 3,000 | 3,000 |
| COUMARIN | 16,000 | 16,000 |
| DAMASCENONE | 2,000 | 2,000 |
| DIHYDRO MYRCENOL | 2,000 | 2,000 |
| DIPROPYLENE GLYCOL | 52,000 | 12,000 |
| FIR BALSAM ABS. 10% DPG | 5,000 | 5,000 |
| FLOROSA | 5,000 | 5,000 |
| GALAXOLIDE 50% IN DPG | 150,000 | 150,000 |
| GERANIOL SUPER | 3,000 | 3,000 |
| GERANYL ACETATE 60 | 2,000 | 2,000 |
| GLOBALIDE® | 210,000 | 210,000 |
| GUAIAC WOOD OIL | 5,000 | 5,000 |
| HEDIONE | 30,000 | 30,000 |
| HEDIONE HC/30 | 60,000 | 60,000 |
| HEXENOL CIS-3 10% DPG | 5,000 | 5,000 |
| ISO E SUPER | 130,000 | 130,000 |
| ISOBUTYL QUINOLINE DL 100% 1% DPG | 5,000 | 5,000 |
| JAVANOL | 3,000 | 3,000 |
| LAVANDINOIL ABRIALIS NAT. | 3,000 | 3,000 |
| LEMON OIL WINTER ITALIE | 15,000 | 15,000 |
| LINALOOL | 15,000 | 15,000 |
| LINALYL ACETATE | 25,000 | 25,000 |
| METHYL IONONE GAMMA PURE / IFF | 3,000 | 3,000 |
| MUSCENONE | 4,000 | 4,000 |
| PATCHOULI OIL DECOL. | 5,000 | 5,000 |
| SANDALWOOD OIL EAST IND. 10% DPG | 5,000 | 5,000 |
| SANDRANOL® | 10,000 | 10,000 |
| TONALIDE | 130,000 | 130,000 |
| VANILLIN | 3,000 | 3,000 |
| VERTOCITRAL 10% DPG | 5,000 | 5,000 |
| VETIVER OIL HAITI | 3,000 | 3,000 |

mit Zusatz von Verbindung 2 od. 3, 4, 5, od. 7 hat die Mischung neben einer intensiveren Ambra- sowie Holznote eine größere Strahlung und wirkt runder und harmonischer.

## Patentansprüche

1. Verbindungen der Formel I: 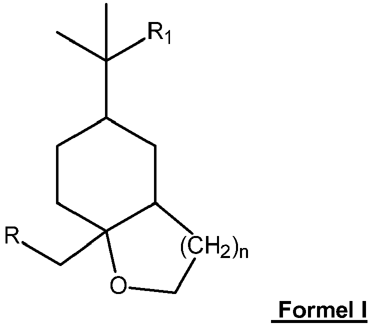 mit
R = -H, -methyl, ethyl, O-methyl oder O-ethyl,
R₁=-H, -methyl, ethyl, O-methyl oder O-ethyl und
n = 1 oder 2.

2. Verbindungen der Formel I nach Anspruch 1 mit
R = -H, -methyl, ethyl, O-methyl oder O-ethyl,
R₁₌-H, -methyl, ethyl, O-methyl oder O-ethyl und
n = 1.

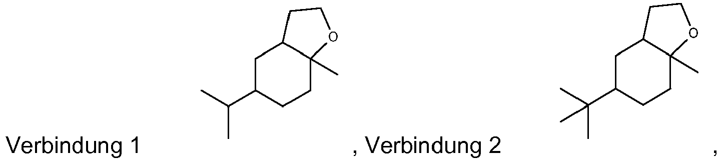

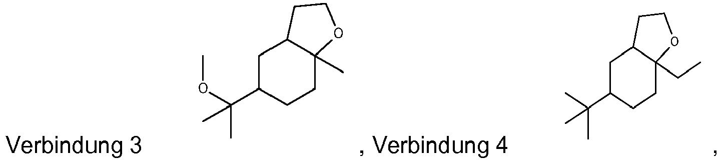

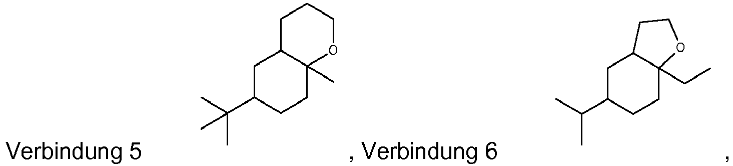

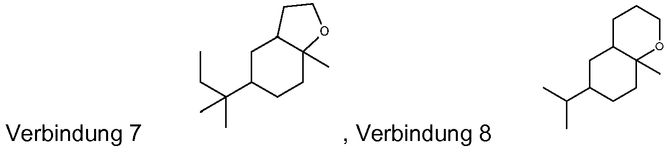

4. Mischungen enthaltend
a) eine oder mehrere Verbindungen der Formel I nach einem der Ansprüche 1 bis 3 und
b) ein oder mehrere Lösungsmittel.

5. Mischungen enthaltend
a) eine oder mehrere Verbindungen der Formel I nach einem der Ansprüche 1 bis 3 und
c) einen oder mehrere Duftstoffe

6. Konsumgüter enthaltend eine oder mehrere Verbindungen der Formel I nach einem der Ansprüche 1 bis 3.

7. Konsumgüter enthaltend Mischungen nach Anspruch 4 oder 5.

8. Verwendung einer oder mehrerer Verbindungen nach einem der Ansprüche 1 bis 3 als Duftstoffe.

9. Verwendung nach Anspruch 8 zur Erzeugung, Vermittlung, Modifizierung oder Verstärkung eines ambrartigen und / oder holzigen Geruchs.

10. Verfahren zur Herstellung von Verbindungen der Formel I nach Anspruch 1 umfassend die Cyclisierung einer Verbindung der Formel II 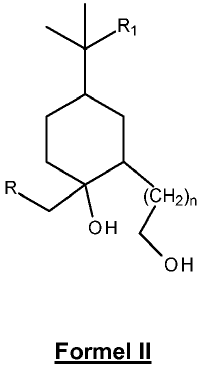 mit
R = -H, -methyl, ethyl, O-methyl, oder O-ethyl,
R₁ = -H, -methyl, ethyl, O-methyl, oder O-ethyl und
n = 1 oder 2.

11. Verfahren zur Herstellung nach Anspruch 10 in Gegenwart eines Sulfonsäurehalogenids und / oder von Tris(dioxa-3,6-heptyl)amin.

12. Verfahren nach Anspruch 10 oder 11 in Gegenwart von Toluol.

13. Verfahren nach Anspruch 10, 11 oder 12, wobei die Temperatur zwischen 30 und 60°C beträgt.

14. Verfahren zur Parfümierung von Konsumgütern umfassend das Hinzufügen einer Verbindung der Formel I nach einem der Ansprüche 1 bis 3 zu Konsumgütern.

## Claims

1. Compounds of the formula I: 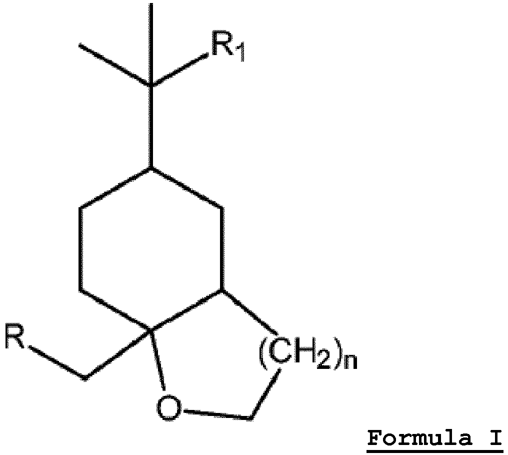 where
R = -H, -methyl, ethyl, 0-methyl or O-ethyl,
R₁ = -H, -methyl, ethyl, O-methyl or O-ethyl and
n = 1 or 2.

2. Compounds of the formula I according to Claim 1 where
R = -H, -methyl, ethyl, O-methyl or O-ethyl,
R₁ = -H, -methyl, ethyl, O-methyl or O-ethyl and
n = 1.

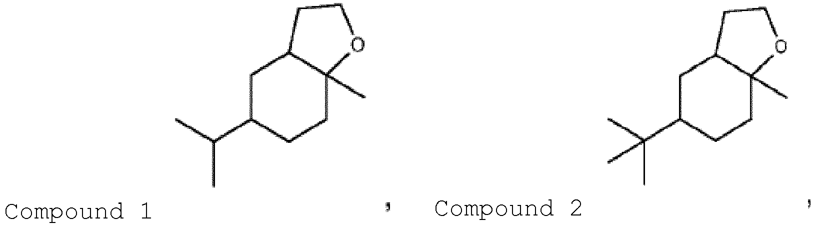

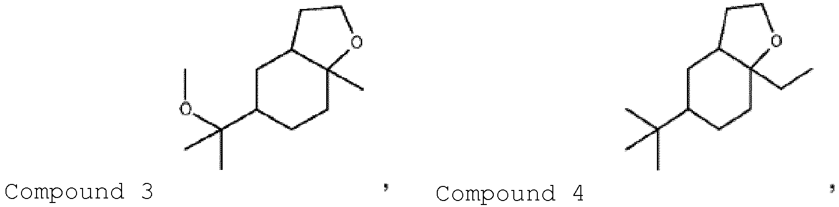

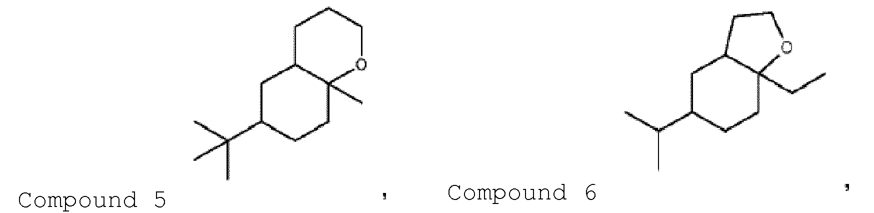

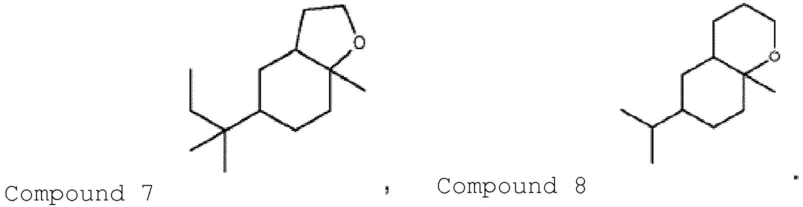

4. Mixtures comprising
a) one or more compounds of the formula I according to one of Claims 1 to 3 and
b) one or more solvents.

5. Mixtures comprising
a) one or more compounds of the formula I according to one of Claims 1 to 3 and
c) one or more fragrances.

6. Consumer goods comprising one or more compounds of the formula I according to one of Claims 1 to 3.

7. Consumer goods comprising mixtures according to Claim 4 or 5.

8. Use of one or more compounds according to one of Claims 1 to 3 as fragrances.

9. Use according to Claim 8 for generating, conveying, modifying or boosting an ambergris-like and/or woody odour.

10. Method for the preparation of compounds of the formula I according to Claim 1, comprising the cyclization of a compound of the formula II 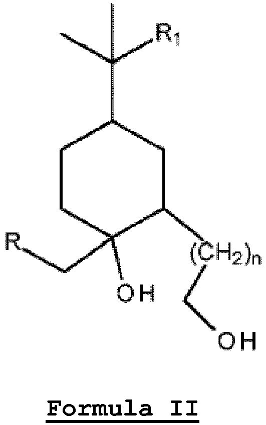 where
R = -H, -methyl, ethyl, O-methyl or O-ethyl,
R¹ = -H, -methyl, ethyl, O-methyl or O-ethyl and
n = 1 or 2.

11. Method for the preparation according to Claim 10 in the presence of a sulphonic acid halide and/or tris(dioxa-3,6-heptyl)amine.

12. Method according to Claim 10 or 11 in the presence of toluene.

13. Method according to Claim 10, 11 or 12, where the temperature is between 30 and 60°C.

14. Method for the perfuming of consumer goods comprising the addition of a compound of the formula I according to one of Claims 1 to 3 to consumer goods.

## Revendications

1. Composés de formule I : 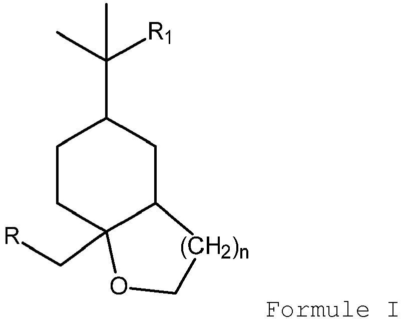 dans laquelle
R = -H, -méthyle, éthyle, O-méthyle ou O-éthyle,
R₁ = -H, -méthyle, éthyle, O-méthyle ou O-éthyle et
n = 1 ou 2.

2. Composés de formule I selon la revendication 1, dans laquelle :
R = -H, -méthyle, éthyle, O-méthyle ou O-éthyle,
R₁ = -H, -méthyle, éthyle, O-méthyle ou O-éthyle et
n = 1.

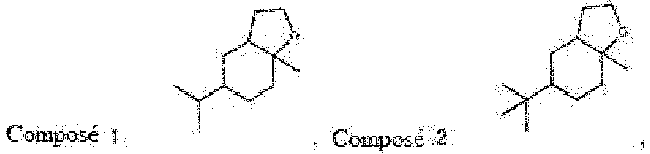

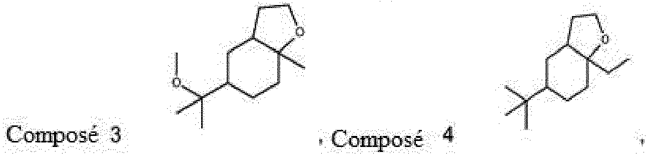

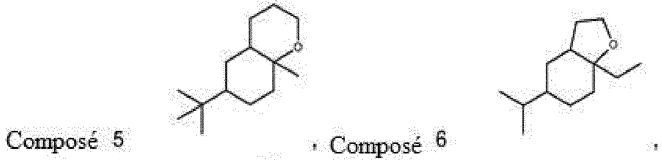

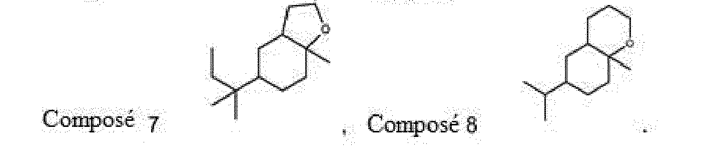

4. Mélanges contenant
a) un ou plusieurs composés de formule I selon l'une quelconque des revendications 1 à 3 et
b) un ou plusieurs solvants.

5. Mélanges contenant
a) un ou plusieurs composés de formule I selon l'une quelconque des revendications 1 à 3 et
c) un ou plusieurs parfums.

6. Bien de consommation contenant un ou plusieurs composés de formule I selon l'une quelconque des revendications 1 à 3.

7. Bien de consommation contenant des mélanges selon la revendication 4 ou 5.

8. Utilisation d'un ou de plusieurs composés selon l'une quelconque des revendications 1 à 3 comme parfum.

9. Utilisation selon la revendication 8 pour obtenir, développer, modifier ou renforcer une odeur de type ambre et/ou de type bois.

10. Procédé pour la préparation de composés de formule I selon la revendication 1, comprenant la cyclisation d'un composé de formule II 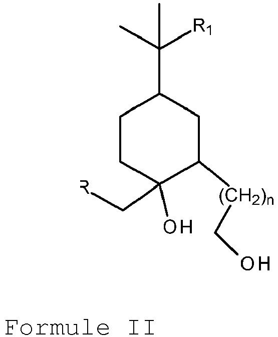 dans laquelle
R = -H, -méthyle, éthyle, 0-méthyle ou 0-éthyle,
R₁ = -H, -méthyle, éthyle, 0-méthyle ou 0-éthyle et
n = 1 ou 2.

11. Procédé pour la préparation selon la revendication 10 en présence d'un halogénure d'acide sulfonique et/ou de tris(dioxa-3,6-heptyl)amine.

12. Procédé selon la revendication 10 ou 11 en présence de toluène.

13. Procédé selon la revendication 10, 11 ou 12, la température étant située entre 30 et 60°C.

14. Procédé pour parfumer des biens de consommation comprenant l'addition d'un composé de formule I selon l'une quelconque des revendications 1 à 3 à des biens de consommation.
